(19) Europäisches Patentamt
European Patent Office
Office européen des brevets

(11) Veröffentlichungsnummer: **0 026 317**
**B1**

(12) # EUROPÄISCHE PATENTSCHRIFT

(45) Veröffentlichungstag der Patentschrift: **16.10.85**

(51) Int. Cl.⁴: **C 07 D 211/90**, A 61 K 31/44

(21) Anmeldenummer: **80104900.8**

(22) Anmeldetag: **16.08.80**

(54) **Optisch aktive 1,4-Dihydropyridine, Verfahren zu ihrer Herstellung und ihre Verwendung als Arzneimittel.**

(30) Priorität: **01.09.79 DE 2935451**

(43) Veröffentlichungstag der Anmeldung: **08.04.81 Patentblatt 81/14**

(45) Bekanntmachung des Hinweises auf die Patenterteilung: **16.10.85 Patentblatt 85/42**

(84) Benannte Vertragsstaaten: **AT BE CH DE FR GB IT LI LU NL SE**

(56) Entgegenhaltungen:
**EP - A - 0 007 293**
**DE - A - 2 117 571**
**DE - A - 2 629 892**
**DE - A - 2 756 226**

**Bearson et al., J.A.C.S. 77 (1955), 451**
**Shibanuma et al., Chem.Pharm. Bull., 28 (9) (1980), 2809-2812**

(73) Patentinhaber: **BAYER AG, Konzernverwaltung RP Patentabteilung, D-5090 Leverkusen 1 Bayerwerk (DE)**

(72) Erfinder: **Wehinger, Egbert, Dr., Donnenberger Strasse 90, D-5620 Velbert 15 (DE)**
Erfinder: **Meyer, Horst, Dr., Theodor-Heuss-Strasse 110, D-5600 Wuppertal 1 (DE)**
Erfinder: **Bossert, Friedrich, Dr., Claudiusweg 1, D-5600 Wuppertal 1 (DE)**
Erfinder: **Vater, Wulf, Dr., Menchendahler Strasse 23, D-5090 Leverkusen 3 (DE)**
Erfinder: **Towart, Robertson, Dr., Claudiusweg 9, D-5600 Wuppertal 1 (DE)**
Erfinder: **Stoepel, Kurt, Dr., In den Birken 69, D-5600 Wuppertal 1 (DE)**
Erfinder: **Kazda, Stanislav, Dr., Pahlkestrasse 55, D-5600 Wuppertal 1 (DE)**

**EP 0 026 317 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft neue optisch aktive 1,4-Dihydropyridine, ein Verfahren zu ihrer Herstellung sowie ihre Verwendung als Arzneimittel, insbesondere als kreislaufbeeinflussende Mittel.

Es ist bereits bekannt geworden, dass bestimmte 1,4-Dihydropyridin-Derivate interessante pharmakologische Eigenschaften aufweisen und insbesondere als kreislaufbeeinflussende Mittel verwendet werden können (vgl. F. Bossert, W. Vater, Naturwissenschaften 58, 578 (1971) und DT-OS 2 117 571). Bei allen bisher bekannten pharmakologisch wirksamen 1,4-Dihydropyridin-Derivaten handelt es sich entweder um achirale Verbindungen oder um Racemformen chiraler Verbindungen.

Es ist weiterhin bekannt, dass die Trennung von Racemformen chiraler, verschiedenartig substituierter 1,4-Dihydropyridin-Derivate bereits versucht wurde, aber die Herstellung und Isolierung von reinen Antipoden bisher noch nicht gelungen ist (J.A. Berson und E. Brown., J. Amer. chem. Soc. 77, 450 (1955)). Die optisch aktiven Antipoden von chiralen 1,4-Dihydropyridinderivaten sind somit ebenso wie das im folgenden beschriebenen Herstellungsverfahren neu und stellen eine Bereicherung der Technik dar.

Gegenstand der vorliegenden Erfindung sind die Antipoden von chiralen 1,4-Dihydropyridincarbonsäureestern mit unterschiedlichen, achiralen Substituenten in 3- und 5-Position der allgemeinen Formel (Ia)

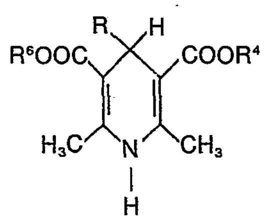

(Ia)

in welcher

R für Nitrophenyl steht,

$R^4$ einen achiralen Alkylrest mit 1–6 Kohlenstoffatomen bedeutet, der gegebenenfalls substituiert ist durch eine Alkoxygruppe mit 1–4 Kohlenstoffatomen, und

$R^6$ für einen achiralen Alkylrest mit 1 bis 6 Kohlenstoffatomen steht.

Gegenstand der vorliegenden Erfindung sind weiterhin einheitlich konfigurierte 1,4-Dihydropyridincarbonsäureester der allgemeinen Formel (Ib)

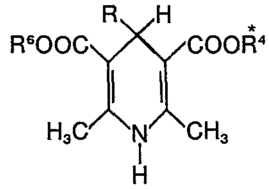

(Ib)

in welcher

R und $R^6$ die in Formel (Ia) angegebene Bedeutung haben, und

$R^{*4}$ für 2-Methoxy-2-phenyl-ethyl steht.

Diese erfindungsgemässen Verbindungen der Formel (Ib) sind wertvolle Zwischenprodukte über welche die Verbindungen der Formel (Ia) in einfacher Weise durch Umesterung des chiralen Substituenten $R^{*4}$ erhalten werden können.

Es wurde gefunden, dass man die optisch aktiven 1,4-Dihydropyridincarbonsäureester der allgemeinen Formeln (Ia) und (Ib) erhält, wenn man optisch aktive Enaminocarbonsäureester der allgemeinen Formel II

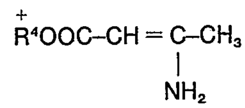

(II)

in welcher $R^{+4}$ die oben angegebene Bedeutung hat, mit Benzyliden-β-dicarbonylverbindungen der allgemeinen Formel III

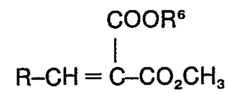

(III)

in welcher R und $R^6$ die oben angegebene Bedeutung haben, zur Reaktion bringt und das entstandene Diastereomerengemisch von Dihydropyridinen der allgemeinen Formel (Ib)

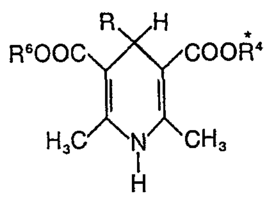

(Ib)

in welcher R, $R^{*4}$ und $R^6$ die oben angegebene Bedeutung haben, nach üblichen Methoden trennt und die erhaltenen Stereoisomere so umestert, dass der chirale Esterrest $R^{*4}$ durch einen achiralen Esterrest $R^4$ ausgetauscht wird.

Die erfindungsgemässen optisch aktiven 1,4-Dihydropyridine der allgemeinen Formel (Ia) besitzen wertvolle pharmakologische Eigenschaften. Aufgrund ihrer kreislaufbeeinflussenden Wirkung können sie als antihypertensive Mittel, als periphere und cerebrale Vasodilatatoren sowie als Coronartherapeutika Verwendung finden. Es wurde gefunden, dass bei den erfindungsgemässen Dihydropyridinen die pharmakologische Wirkung von der Konfiguration abhängt und dass einer der Antipoden immer eine deutlich bessere Wirkung als das entsprechende Racemat zeigt. Völlig unerwartet war darüber hinaus, dass die pharmakologische Wirkung nicht durch die unterschiedlichen Konfigurationen im chiralen Esterrest beeinflusst wird, sondern einzig durch die Konfiguration des Kohlenstoffatoms in 4-Position des Dihydropyridinringes. Durch diesen unerwarteten Befund wird der Fachmann in die Lage versetzt, die bereits bekannten wertvollen Eigenschaften von Dihydropyridinen spezifischer einzusetzen, neue galenische Zubereitungen mit geringerem Wirkstoffgehalt herzustellen und unerwünschte pharmakologische Nebenwirkungen zu reduzieren.

Die erfindungsgemässen neuen optisch aktiven 1,4-Dihydropyridine stellen somit eine Bereicherung der Pharmazie dar.

Gemäss dem erfindungsgemässen Verfahren wird zunächst nach einer üblichen 1,4-Dihydropyridin-Synthese ein Dihydropyridinester der Formel (Ib) synthetisiert, der eine chirale Alkoholkomponente ($-OR^{*4}$) besitzt. Dabei bilden sich aufgrund der beiden möglichen, entgegengesetzten Konfigurationen am $C_4$-Atom des 1,4-Dihydropyridinringes 2 Diastereomere. Diese werden nach üblichen Methoden getrennt und anschliessend die chirale Alkoholkomponente durch eine achirale Alkoholkomponente ($-OR^4$) ersetzt, so dass Verbindungen der Formel (Ia) entstehen.

Bei diesem Verfahren lassen sich als Alkoholkomponenten $-OR^{*4}$ sowohl rechtsdrehende als auch linksdrehende Alkoholreste verwenden, so dass im folgenden auf die Spezifizierung der Konfiguration im Rest $-OR^{*4}$ nicht näher eingegangen werden braucht.

Die als Ausgangsstoffe verwendeten β-Aminocarbonsäureester der Formel II und die Ylidene der Formel III sind bekannt oder können nach bekannten Methoden hergestellt werden (vergl. DE-OS 2 117 571). Die bei der Reaktion als Zwischenprodukte entstehenden Verbindungen der Formel Ib sind neu.

Als Beispiele seien genannt:

1.4-Dihydro-2.6-dimethyl-4-(3′-nitrophenyl)-pyridin-3.5-dicarbonsäure-methyl-(β-methoxy-β-phenyl-ethyl)-ester

1.4-Dihydro-2.6-dimethyl-4-(2′-nitrophenyl)-pyridin-3.5-dicarbonsäure-ethyl-(β-methoxy-β-phenyl)-ethyl)-ester

1.4-Dihydro-2.6-dimethyl-4-(3′-nitrophenyl)-pyridin-3.5-dicarbonsäure-isopropyl-(β-methoxy-β-phenyl-ethyl)-ester

1.4-Dihydro-2.6-dimethyl-4-(2′-nitrophenyl)-pyridin-3.5-dicarbonsäure-isobutyl-(β-methoxy-β-phenyl-ethyl)-ester.

Die verfahrensgemäss erhältlichen Verbindungen der Formel (Ib) unterscheiden sich als Diastereomere in ihren physikalischen und chemischen Eigenschaften und können daher mit Hilfe bekannter Methoden voneinander getrennt werden. Als Trennmethoden seien vorzugsweise genannt: Umkristallisation aus inerten Lösungsmitteln, Dünnschichtchromatographie, Säulenchromatographie oder Hochdruckflüssigkeitschromatographie.

Die getrennten, konfigurativ einheitlichen 1,4-Dihydropyridin-Derivate der Formel (Ib) sind als Zwischenprodukte zur einfachen Herstellung von Verbindungen der Formel Ia geeignet und stellen bereits wertvolle pharmakologische Wirkstoffe dar. Die eventuell erforderliche Umesterung der Verbindungen (Ib) erfolgt vorzugsweise über alkalische Alkanolyse, gegebenenfalls in Gegenwart eines inerten zusätzlichen Lösungsmittels unter Verwendung von $R^4-O^0$ als alkoholischen Agens, wobei $R^4$ die oben angegebene Bedeutung besitzt.

Als Lösungsmittel für diese Umesterung kommen alle inerten organischen Lösungsmittel oder Gemische derselben in Frage. Hierzu gehören vorzugsweise Ether wie Dioxan, Tetrahydrofuran, Glykolmonomethylether, Glykoldimethylether oder Dimethylformamid, Dimethylsulfoxid, Acetonitril, Pyridin oder Hexamethylphosphorsäuretriamid.

Die Reaktionstemperaturen können in einem grösseren Bereich variiert werden. Im allgemeinen arbeitet man zwischen 20 und 150 °C, vorzugsweise bei ca. 50 bis 100 °C.

Die Umsetzung kann bei Normaldruck, aber auch bei erhöhtem Druck durchgeführt werden. Im allgemeinen arbeitet man unter Normaldruck.

Als Alkoholyseagentien kommen vorzugsweise Alkalialkoholate, wie Natrium- oder Kaliumalkoholate in Betracht. Diese werden bei der Durchführung der Alkoholyse jeweils in molaren Mengen in geringem Überschuss eingesetzt.

Ausser den unten angeführten Herstellungsbeispielen seien folgende, optisch aktive erfindungsgemässen Verbindungen genannt:

1.4-Dihydro-2.6-dimethyl-4-(3′-nitrophenyl)-pyridin-3.5-dicarbonsäure-methyl-ethylester

1.4-Dihydro-2.6-dimethyl-4-(3′-nitrophenyl)-pyridin-3.5-dicarbonsäure-methyl-propylester

1.4-Dihydro-2.6-dimethyl-4-(3′-nitrophenyl)-pyridin-3.5-dicarbonsäure-methyl-(2-methoxy-ethyl)-ester

1.4-Dihydro-2.6-dimethyl-4-(2′-nitrophenyl)-pyridin-3.5-dicarbonsäure-methyl-ethylester

1.4-Dihydro-2.6-dimethyl-4-(2′-nitrophenyl)-pyridin-3.5-dicarbonsäure-methyl-isopropylester

1.4-Dihydro-2.6-dimethyl-4-(2′-nitrophenyl)-pyridin-3.5-dicarbonsäure-methyl-isobutylester

1.4-Dihydro-2.6-dimethyl-4-(2′-nitrophenyl)-pyridin-3.5-dicarbonsäure-ethyl-isobutylester

1.4-Dihydro-2.6-dimethyl-4-(3′-nitrophenyl)-pyridin-3.5-dicarbonsäure-isopropyl-(2-propoxyethyl)-ester.

Die erfindungsgemässen Verbindungen der Formel (Ia) sind als Arzneimittel, insbesondere als gefäss- und kreislaufbeeinflussende Wirkstoffe verwendbar. Sie haben ein breites und vielseitiges pharmakologisches Wirkungsspektrum. Im einzelnen konnten im Tierexperiment folgende Hauptwirkungen nachgewiesen werden:

1. Die Verbindungen bewirken bei parenteraler, oraler und perlingualer Zugabe eine deutliche und lang anhaltende Erweiterung der Coronargefässe. Diese Wirkung auf die Coronargefässe wird durch einen gleichzeitigen nitritähnlichen herzentlastenden Effekt verstärkt.

Sie beeinflussen bzw. verändern den Herzstoffwechsel im Sinne der Energieersparnis.

2. Die Erregbarkeit des Reizbildungs- und Erregungsleitungssystem innerhalb des Herzens wird herabgesetzt, so dass eine in therapeutischen Dosen nachweisbare Antiflimmerwirkung resultiert.

3. Der Tonus der glatten Muskulatur der Gefässe wird unter der Wirkung der Verbindungen stark vermindert. Diese gefässspasmolytische Wirkung kann im gesamten Gefässsystem stattfinden, oder sich mehr oder weniger isoliert in umschriebenen

Gefässgebieten, wie z.B. dem Zentralnervensystem, insbesondere im cerebralen Bereich manifestieren.

4. Die Verbindungen senken den Blutdruck von normotonen und hypertonen Tieren und können somit als antihypertensive Mittel verwendet werden.

5. Die Verbindungen haben stark muskulärspasmolytische Wirkung, dies an der glatten Muskulatur des Magens, Darmtraktes, des Urogenitaltraktes und des Respirationssystems deutlich werden.

Die erfindungsgemässen Verbindungen eignen sich aufgrund dieser Eigenschaften besonders zur Prophylaxe und Therapie der akuten und chronischen ischämischen Herzkrankheit im weitesten Sinne, zur Therapie des Hochdrucks sowie zur Behandlung von cerebralen und peripheren Durchblutungsstörungen.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nichttoxischer pharmazeutisch geeigneter Trägerstoffe oder Lösungsmittel. Hierbei soll die therapeutisch wirksame Verbindung jeweils in einer Konzentration von etwa 0,5 bis 90 Gewichtsprozent der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Falle der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nichttoxische organische Lösungsmittel, wie Paraffine (z.B. Erdölfraktionen), pflanzliche Öle (z.B. Erdnuss-/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle, (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker), Emulgiermittel, wie nichtionogene und anionische Emulgatoren (z.B. Polyoxyethylen-Fettsäure-Ester, Polyoxyethylen-Fett-alkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich ausser den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wässriger Suspensionen und/oder Elixieren, die für orale Anwendungen gedacht sind, können die Wirkstoffe ausser dem den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,05 bis 1 mg/kg Körpergewicht pro Tag zur Erzielung wirksamer Ergebnisse zu verabreichen, und bei oraler Applikation beträgt die Dosierung etwa 0,05 bis 20 mg/kg, vorzugsweise 0,1 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres, bzw. der Art des Applikationsweges, aber auch aufgrund der Tierart und deren individuellem Verhalten gegenüber dem Medikament bzw. der Art von dessen Formulierung und dem Zeitpunkt bzw. Intervall zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation grösserer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäss gelten hierbei auch die obigen Ausführungen.

Die optische Reinheit der gemäss den folgenden Herstellungsbeispielen erhaltenen Verbindungen der Formel (Ia) und (Ib) ebenso wie der jeweiligen Ausgangs- und Zwischenprodukte wurde protonenresonanzspektroskopisch durch Zugabe von (chiralen) Lanthaniden-Shiftagentien überprüft und sichergestellt.

Herstellungsbeispiele
(Verbindungen der Formel (Ib)

A) Darstellung von
(+)-1,4-Dihydro-2.6-dimethyl-4-(3'-nitrophenyl)-pyridin-3.5-dicarbonsäure-isopropyl-(2-methoxy-2-phenylethyl)-ester
(Verbindung A)

(+)

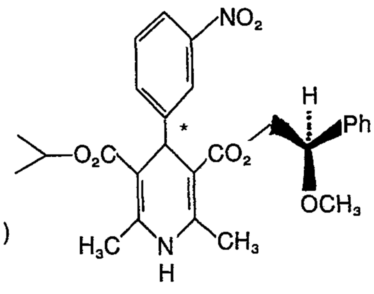

Eine Lösung von 14,5 g (96 mMol) 3-Nitrobenzaldehyd, 13,7 g (96 mMol) β-Aminocrotonsäureisopropylester und 22,8 g (96 mMol) Acetessigsäure-(2-methoxy-2-phenylethyl)-ester in 150 ml Isopropanol wurde 12 Stunden unter Stickstoff zum Sieden erhitzt.

Anschliessend wurde das Lösungsmittel im Vakuum abdestilliert und der ölige Rückstand mit wenig Ether verrieben, wobei sich das Reaktionsprodukt teilweise verfestigte. Die ausgefallenen Kristalle wurden abgesaugt und aus Ethanol umkristallisiert.

Schmelzpunkt: Fp = 173 °C, Ausbeute: 19 g (40%). Diese kristalline Fraktion in konfigurativ einheitlich, die spezifische Drehung beträgt: $[\alpha]_D^{20} = +53.82°$ (c = 1.1% w/v*, Ethanol).

B) Darstellung von
(−)-1.4-Dihydro-2.6-dimethyl-4-(3′-nitrophenyl)-pyridin-3.5-dicarbonsäure-isopropyl-(2-methoxy-2-phenylethyl)-ester
(Verbindung B)

(−) 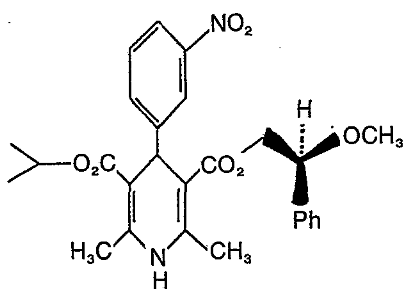

Eine Lösung von 27,7 g (0,1 Mol) 3′-Nitrobenzylidenacetessigsäureisopropylester und 23,5 g (0,1 Mol) β-Aminocrotonsäure-(2-methoxy-2-phenylethyl)-ester in 160 ml Methanol wurde 12 Stunden unter Stickstoff zum Sieden erhitzt. Anschliessend wurde das Lösungsmittel im Vakuum abdestilliert und der ölige Rückstand mit wenig Ether verrieben. Das Reaktionsprodukt verfestigte sich dabei, es wurde abgesaugt und aus Ethanol umkristallisiert.

Schmelzpunkt: Fp = 173 °C, Ausbeute: 11,8 g (24%).

Das Produkt ist konfigurativ einheitlich, die spezifische Drehung beträgt: $[\alpha]_D^{20} = -53.3°$ (c = 1.04% w/v, Ethanol).

Beispiel 1 (Verbindungen der Formel (Ia))
(+)-1.4-Dihydro-2.6-dimethyl-4-(3′-nitrophenyl)-pyridin-3.5-dicarbonsäure-isopropyl-methylester

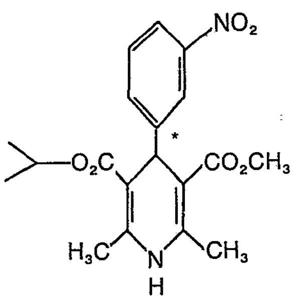 (+)

* w/v = weight per Volume.

19 g (38 mMol)
(+)-1.4-Dihydro-2.6-dimethyl-4-(3′-nitrophenyl)-pyridin-3.5-dicarbonsäure-isopropyl-(2-methoxy-2-phenylethyl)-ester
(Verbindung A)
wurde in einer Lösung von 0,87 g (38 mMol) Natrium in 50 ml Methanol und 50 ml 1.2-Dimethoxyethan 5 Stunden unter Stickstoff zum Sieden erhitzt. Anschliessend wurde die Lösung im Vakuum bis zur Hälfte eingeengt und mit verdünnter Chlorwasserstoffsäure angesäuert. Nach Zugabe eines gleichen Teiles Wasser wurde die Mischung mehrmals mit Methylenchlorid extrahiert und die Extrakte nach Trocknen über Natriumsulfat im Vakuum eingeengt. Der feste Rückstand wurde aus Methanol umkristallisiert (Rohausbeute 5,5 g (39%), Fp: 134–136 °C) und anschliessend mit Hilfe der Hochdruckflüssigkeitschromatographie an einer präparativen RP8-Säule (10 µ), Innendurchmesser 16 mm, Länge 250 mm, unter Verwendung von Acetonitril/Wasser = 45/55 als Eluierungsmittel gereinigt.

Schmelzpunkt: Fp = 136 °C.

Spezifische Drehung: $[\alpha]_D^{20} = +24.97$ (c = 0,93% w/v, Ethanol).

Beispiel 2
(−)-1.4-Dihydro-2.6-dimethyl-4-(3′-nitrophenyl)-pyridin-3.5-dicarbonsäure-isopropyl-methylester

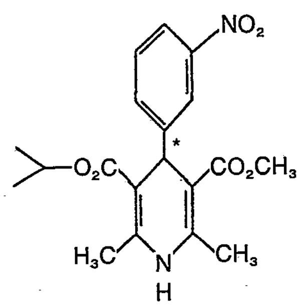 (−)

16,5 g (33.4 mMol)
(−)-1.4-Dihydro-2.6-dimethyl-4-(3′-nitrophenyl)-pyridin-3.5-dicarbonsäure-isopropyl-(2-methoxy-2-phenylethyl)-ester
(Verbindung B)
wurden in einer Lösung von 0,87 g (38 mMol) Natrium in 100 ml Methanol 24 Stunden unter Stickstoff zum Sieden erhitzt. Nach dem Erkalten wurde das Lösungsmittel im Vakuum etwa bis zur Hälfte eingeengt und mit verdünnter Chrowasserstoffsäure angesäuert. Nach Zugabe eines gleichen Teiles Wasser wurde die Mischung mehrmals mit Methylenchlorid extrahiert und die Extrakte nach Trocknen über Natriumsulfat im Vakuum eingeengt. Der Rückstand kristallisierte durch, wurde abgesaugt, aus Methanol umkristallisiert (Rohausbeute 4,2 g (34%), Fp: 132–135 °C) und mit Hilfe der Hochdruckflüssigkeitschromatographie, wie unter Beispiel 1 beschrieben, gereinigt.

Schmelzpunkt: Fp = 136 °C.

Spezifische Drehung: $[\alpha]_D^{20} = -24.60°$ (c = 1,07% w/v, Ethanol).

Beispiel 3

(+)-1.4-Dihydro-2.6-dimethyl-4-(3'-nitrophenyl)-pyridin-3.5-dicarbonsäure-ethyl-isopropylester

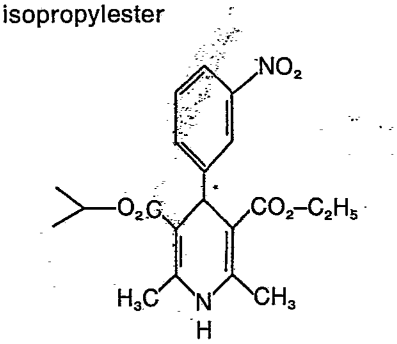

(+)

19 g (38 mMol) (+)-1.4-Dihydro-2.6-dimethyl-4-(3'-nitrophenyl)-pyridin-3.5-dicarbonsäure-isopropyl-(2-methoxy-2-phenylethyl)-ester (Verbindung A) wurden in einer Lösung von 0,87 g (38 mMol) Natrium in 100 ml Ethanol 8 Stunden unter Stickstoff zum Sieden erhitzt. Anschliessend wurde das Lösungsmittel im Vakuum abdestilliert, der Rückstand in Wasser aufgenommen, die Mischung mit verdünnter Chlorwasserstoffsäure angesäuert und mehrmals mit Methylenchlorid extrahiert. Nach Trocknen über Natriumsulfat wurden die Extrakte im Vakuum eingeengt. Der Rückstand kristallisierte durch, wurde abgesaugt, aus Methanol umkristallisiert (Rohausbeute 4,1 g (28%), Fp: 143–146 °C) und mit Hilfe der Hochdruckflüssigkeitschromatographie, wie unter Beispiel 1 beschrieben, gereinigt.

Schmelzpunkt: Fp = 140 °C.

Spezifische Drehung: $[\alpha]_D^{20}$ = + 4.61 (c = 0,46% w/v, Ethanol).

Beispiel 4

(−)-1.4-Dihydro-2.6-dimethyl-4-(3'-nitrophenyl)-pyridin-3.5-dicarbonsäure-ethyl-isopropylester

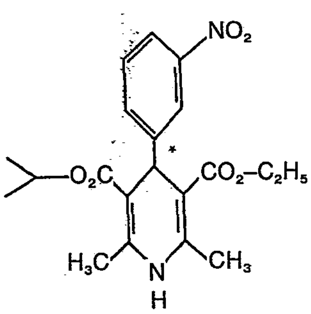

(−)

18,6 g (37,6 mMol) (−)-1.4-Dihydro-2.6-dimethyl-4-(3'-nitrophenyl)-pyridin-3.5-dicarbonsäure-isopropyl-(2-methoxy-2-phenylethyl)-ester (Verbindung B) wurden in einer Lösung von 0,86 g (37,6 mMol) Natrium in 100 ml Ethanol 8 Stunden unter Stickstoff zum Sieden erhitzt. Anschliessend wurde die Lösung im Vakuum etwa bis zur Hälfte eingeengt, mit verdünnter Chlorwasserstoffsäure angesäuert und nach Zugabe eines gleichen Teiles Wasser mehrmals mit Methylenchlorid extrahiert. Nach Trocknen über Natriumsulfat wurden die Extrakte im Vakuum eingeengt. Der zunächst ölig anfallende Rückstand kristallisierte bald durch, er wurde abgesaugt, aus Methanol umkristallisiert (Rohausbeute 2,8 g (19,2%), Fp: 146–149 °C) und mit Hilfe der Hochdruckflüssigkeitschromatographie, wie unter Beispiel 1 beschrieben, gereinigt.

Schmelzpunkt: Fp = 140°.

Spezifische Drehung: $[\alpha]_D^{20}$ = − 4.75 (c = 0,51% w/v, Ethanol).

Beispiel 5

(+)-1.4-Dihydro-2.6-dimethyl-4-(3'-nitrophenyl)-pyridin-3.5-dicarbonsäure-isopropyl-(2-methoxyethyl)-ester

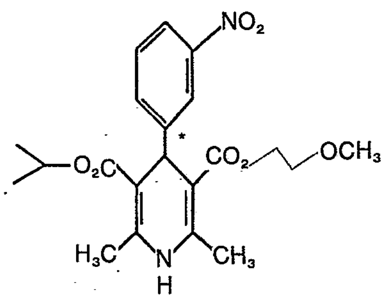

(+)

24,7 g (50 mMol) (+)-1.4-Dihydro-2.6-dimethyl-4-(3'-nitrophenyl)-pyridin-3.5-dicarbonsäure-isopropyl-(2-methoxy-2-phenylethyl)-ester (Verbindung A) wurden in einer Lösung von 1,4 g (61 mMol) Natrium in 230 ml frisch destillierten Glykolmonomethylethers 8 Stunden unter Stickstoff bei 85 °C gerührt. Nach dem Erkalten wurde das Lösungsmittel im Vakuum abdestilliert, der Rückstand in Wasser aufgenommen, die Mischung mit verdünnter Chlorwasserstoffsäure angesäuert und mehrmals mit Methylenchlorid extrahiert. Die organischen Extrakte wurden nach Trocknen über Natriumsulfat eingeengt, der ölige Rückstand mit wenig Ether verrieben, wobei die Substanz schnell durchkristallisierte. Das feste Reaktionsprodukt wurde abgesaugt (Rohausbeute 15,1 g (72%), Fp: 126–129°C) und zweimal aus Ethanol umkristallisiert.

Schmelzpunkt: Fp = 134 °C.

Spezifische Drehung: $[\alpha]_D^{20}$ = + 17.10° (c = 0,96% w/v, Ethanol).

Beispiel 6

(−)-1.4-Dihydro-2.6-dimethyl-4-(3'-nitrophenyl)-pyridin-3,5-dicarbonsäure-isopropyl-(2-methoxy-ethyl)-ester

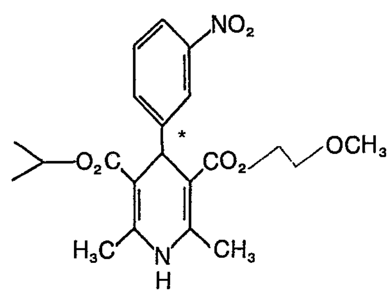

(−)

8,2 g (16,6 mMol)
(−)-1.4-Dihydro-2.6-dimethyl-4-(3′-nitrophenyl)-pyridin-3.5-dicarbonsäure-isopropyl-(2-methoxy-2-phenylethyl)-ester
(Verbindung B)
wurde in einer Lösung von 0,5 g (21,8 mMol) Natrium in 80 ml frisch destillierten Glykolmonomethylethers 8 Stunden unter Stickstoff bei 85 °C gerührt. Nach dem Erkalten wurde das Lösungsmittel im Vakuum abdestilliert, der Rückstand in Wasser aufgenommen, die Mischung mit verdünnter Chlorwasserstoffsäure angesäuert und mehrmals mit Methylenchlorid extrahiert. Die organischen Extrakte wurden nach Trocknen über Natriumsulfat eingeengt. Der ölige Rückstand kristallisierte bald durch, wurde nach Zugabe von wenig Ether abgesaugt (Rohausbeute 5,1 g (73%)) und zweimal aus Ethanol umkristallisiert.

Schmelzpunkt: Fp = 134 °C.

Spezifische Drehung: $[\alpha]_D^{20} = -16.9$ (c = 1,5% w/v, Ethanol).

**Patentansprüche**

1. Antipoden von chiralen 1,4-Dihydropyridincarbonsäureestern mit unterschiedlichen achiralen Substituenten in 3- und 5-Position der allgemeinen Formel Ia

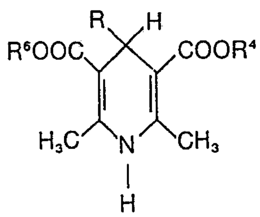

(Ia)

in welcher
R für Nitrophenyl steht,
$R^4$ einen achiralen Alkylrest mit 1–6 Kohlenstoffatomen bedeutet, der gegebenenfalls substituiert ist durch eine Alkoxygruppe mit 1–4 Kohlenstoffatomen und
$R^6$ für einen achiralen Alkylrest mit 1 bis 6 Kohlenstoffatomen steht.

2. 1,4-Dihydropyridincarbonsäureester der allgemeinen Formel (Ib)

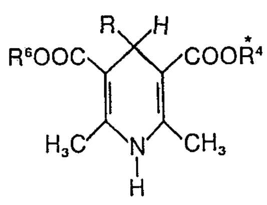

(Ib)

in welcher R und $R^6$ die in Anspruch 1 angegebene Bedeutung haben, und
$R^{*4}$ für 2-Methoxy-2-phenylethyl steht.

3. Verfahren zur Herstellung von optisch aktiven 1,4-Dihydropyridincarbonsäureestern der allgemeinen Formeln (Ia)

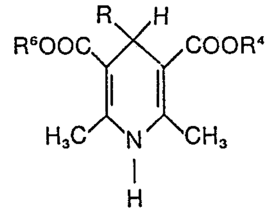

(Ia)

in welcher
R für Nitrophenyl steht,
$R^4$ einen achiralen Alkylrest mit 1–6 Kohlenstoffatomen bedeutet, der gegebenenfalls substituiert ist durch eine Alkoxygruppe mit 1–4 Kohlenstoffatomen und
$R^6$ für einen achiralen Alkylrest mit 1 bis 6 Kohlenstoffatomen steht,
und (Ib)

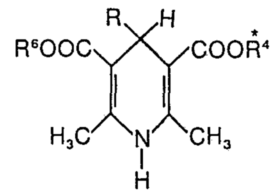

(Ib)

in welcher R und $R^6$ die oben angegebene Bedeutung haben und $R^{*4}$ für 2-Methoxy-2-phenylethyl steht, dadurch gekennzeichnet, dass man optisch aktive Enaminocarbonsäureester der allgemeinen Formel II

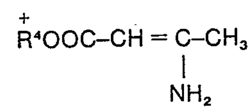

(II)

in welcher $R^{+4}$ die oben angegebene Bedeutung hat, mit Benzyliden-β-dicarbonylverbindungen der allgemeinen Formel III

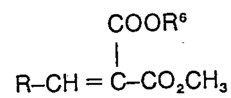

(III)

in welcher R und $R^6$ die oben angegebene Bedeutung haben, zur Reaktion bringt und das entstandene Diastereomerengemisch von Dihydropyridinen der allgemeinen Formel (Ib)

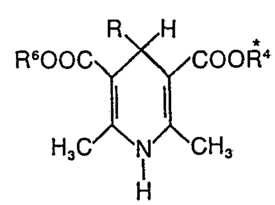

(Ib)

in welcher R, $R^{*4}$ und $R^6$ die oben angegebene Bedeutung haben, nach üblichen Methoden trennt und die erhaltenen Stereoisomere so umestert, dass der chirale Esterrest $R^{+4}$ durch einen achiralen Esterrest $R^4$ ausgetauscht wird.

4. Verwendung von Verbindungen der allgemeinen Formel (Ib) gemäss Anspruch 2, zur Herstellung von Verbindungen der allgemeinen Formel (Ia) gemäss Anspruch 1.

5. Arzneimittel enthaltend mindestens eine Verbindung der allgemeinen Formel (Ia) gemäss Anspruch 1.

6. Verfahren zur Herstellung von Arzneimitteln, dadurch gekennzeichnet, dass man Verbindungen der allgemeinen Formel Ia gemäss Anspruch 3 gegebenenfalls zusammen mit üblichen Hilfs- und Trägerstoffen in eine geeignete Applikationsform überführt.

7. Verbindungen der allgemeinen Formel Ia gemäss Anspruch 1 zur Verwendung bei der Bekämpfung von Erkrankungen.

## Claims

1. Antipodes of chiral 1,4-dihydropyridinecarboxylic acid esters with different achiral substituents in the 3-position and 5-position, of the general formula Ia

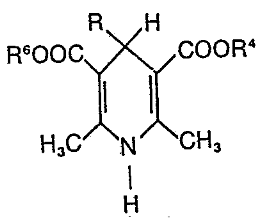
(Ia)

in which

R represents nitrophenyl,

$R^4$ denotes an achiral alkyl radical with 1–6 carbon atoms, which is optionally substituted by an alkoxy group with 1–4 carbon atoms and

$R^6$ represents an achiral alkyl radical with 1 to 6 carbon atoms.

2. 1,4-Dihydropyridinecarboxylic acid esters of the general formula (Ib)

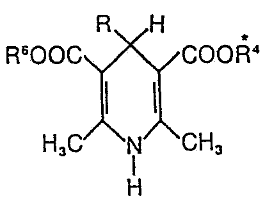
(Ib)

in which

R and $R^6$ have the meaning given in Claim 1 and

$R^{*4}$ represents 2-methoxy-2-phenylethyl.

3. Process for the preparation of optically active 1,4-dihydropyridinecarboxylic acid esters of the general formulae (Ia)

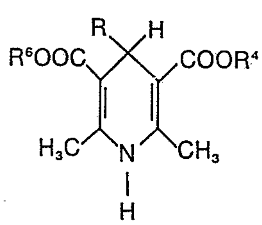
(Ia)

in which

R represents nitrophenyl,

$R^4$ denotes an achiral alkyl radical with 1–6 carbon atoms, which is optionally substituted by an alkoxy group with 1–4 carbon atoms and

$R^6$ represents an achiral alkyl radical with 1 to 6 carbon atoms, and (Ib)

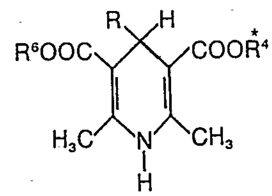
(Ib)

in which

R and $R^6$ have the meaning given above and

$R^{*4}$ represents 2-methoxy-2-phenylethyl, characterised in that optically active enaminocarboxylic acid esters of the general formula II

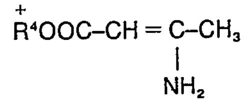
(II)

in which

$R^{+4}$ has the meaning given above,

are reacted with benzylidene-β-dicarbonyl compounds of the general formula III

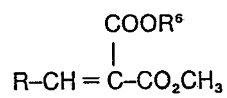
(III)

in which

R and $R^6$ have the meaning given above,

and the resulting diastereomer mixture of dihydropyridines of the general formula (Ib)

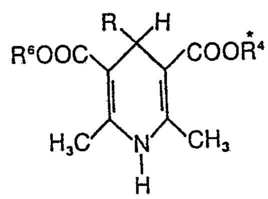
(Ib)

in which

R, $R^{*4}$ and $R^6$ have the meaning given above, is separated by customary methods and the stereoisomers obtained are trans-esterified in such a manner than the chiral ester radical $R^{+4}$ is replaced by an achiral ester radical $R^4$.

4. Use of compounds of the general formula (Ib) according to Claim 2 for the preparation of compounds of the general formula (Ia) according to Claim 1.

5. Medicaments containing at least one compound of the general formula (Ia) according to Claim 1.

6. Process for the preparation of medicaments, characterised in that compounds of the general formula Ia according to Claim 3 are converted into a suitable form of administration, if appropriate together with customary auxiliaries and excipients.

7. Compounds of the general formula Ia according to Claim 1 for use in combating illnesses.

**Revendications**

1. Antipodes d'esters d'acides 1,4-dihydropyridine-carboxyliques chiraux comportant des substituants achiraux différents dans les positions 3 et 5 de la formule générale Ia:

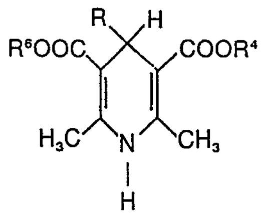

(Ia)

dans laquelle

R représente un groupe nitrophényle,

$R^4$ représente un groupe alkyle achiral contenant 1 à 6 atomes de carbone et éventuellement substitué par un groupe alcoxy contenant 1 à 4 atomes de carbone, et

$R^6$ représente un groupe alkyle achiral contenant 1 à 6 atomes de carbone.

2. Esters d'acides 1,4-dihydropyridine-carboxyliques de formule générale (Ib):

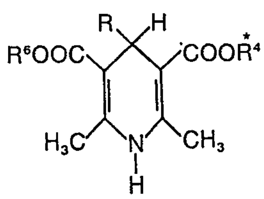

(Ib)

dans laquelle R et $R^6$ ont les significations indiquées dans la revendication 1 et

$R^{*4}$ représente un groupe 2-méthoxy-2-phényléthyle.

3. Procédé de préparation d'esters d'acides 1,4-dihydropyridine-carboxyliques optiquement actifs répondant aux formules générales (Ia).

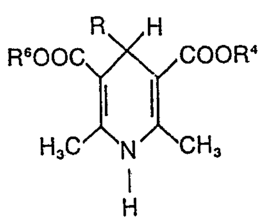

(Ia)

dans laquelle

R représente un groupe nitrophényle,

$R^4$ représente un groupe alkyle achiral contenant 1 à 6 atomes de carbone et éventuellement substitué par un groupe alcoxy contenant 1 à 4 atomes de carbone, et

$R^6$ représente un groupe alkyle achiral contenant 1 à 6 atomes de carbone,

et (Ib):

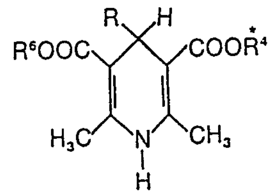

(Ib)

dans laquelle R et $R^6$ ont les significations indiquées ci-dessus et $R^{*4}$ représente un groupe 2-méthoxy-2-phényléthyle,

caractérisé en ce qu'on fait réagir des esters d'acides énamino-carboxyliques optiquement actifs de formule générale (II):

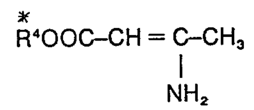

(II)

dans laquelle $R^{*4}$ a la signification indiquée ci-dessus, avec des composés benzylidène-β-dicarbonyle de formule générale III:

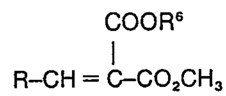

(III)

dans laquelle R et $R^6$ ont les significations indiquées ci-dessus, et on sépare le mélange obtenu de diastéréoisomères de dihydropyridines de formule générale (Ib):

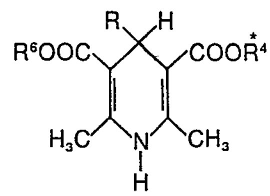

(Ib)

dans laquelle R, $R^{*4}$ et $R^6$ ont les significations indiquées ci-dessus,

selon des méthodes habituelles et on transestérifie les stéréoisomères ainsi obtenus de telle sorte que le radical ester chiral $R^{*4}$ soit remplacé par un radical ester achiral $R^4$.

4. Utilisation de composés de formule générale (Ib) selon la revendication 2 pour la préparation de composés de formule générale (Ia) selon la revendication 1.

5. Médicaments contenant au moins un composé de formule générale (Ia) selon la revendication 1.

6. Procédé de préparation de médicaments, caractérisé en ce qu'on transforme des composés de formule générale Ia selon la revendication 3, éventuellement avec des substances auxiliaires et des substances supports habituelles, sous une forme d'application appropriée.

7. Composés de formule générale Ia selon la revendication 1, destinés à être utilisés pour combattre des maladies.